# EUROPÄISCHE PATENTANMELDUNG

(11) **EP 1 106 141 A2**
(43) Veröffentlichungstag der Anmeldung: **13.06.2001**
(21) Anmeldenummer: 00204216.6
(22) Anmeldetag: 28.11.2000
(51) Int. Cl.: A61B 6/00

(54) **Röntgeneinrichtung mit einem Roboterarm**

(30) Priorität: 07.12.1999 DE 19958864
(71) Anmelder: Philips Corporate Intellectual Property GmbH, 52064 Aachen (DE); Koninklijke Philips Electronics N.V., 5621 BA Eindhoven (NL)
(72) Erfinder: Rasche, Dr. Volker, Philips Corp. Int. Prop. GmbH, 52064 Aachen (DE); Schomberg, Dr. H., Philips Corp. Int. Prop. GmbH, 52064 Aachen (DE); Klotz, Erhard, Philips Corp. Int. Prop. GmbH, 52064 Aachen (DE)
(74) Vertreter: Volmer, Georg, Dipl.-Ing.

(57) **Zusammenfassung**

Die Erfindung betrifft eine Röntgeneinrichtung mit einer Röntgenquelle (2) und einem Röntgendetektor (3), welche an jeweils einem Ende einer gemeinsamen Haltevorrichtung (12) angeordnet sind, wobei die Haltevorrichtung (12) durch eine Tragvorrichtung (18) mit dem Raum (9) verbunden ist. Um solche Röntgeneinrichtungen, die vielfach benutzt werden und bei denen die Haltevorrichtung (12) im allgemeinen als C-Bogen ausgestaltet ist, flexibler zu gestalten, wobei eine hohe Positionsgenauigkeit beibehalten werden soll, ist erfindungsgemäß vorgesehen, die Tragvorrichtung (18) aus mehreren gelenkig seriell miteinander verbundenen Traggliedern (15,16,17) aufzubauen. Insbesondere soll als Tragvorrichtung ein serieller Manipulator, beispielsweise ein konventioneller Roboterarm verwendet werden.

## Beschreibung

Die Erfindung betrifft eine Röntgeneinrichtung gemäß dem Oberbegriff des Anspruchs 1.

Derartige Röntgeneinrichtungen sind vielfach bekannt und benutzt. Dabei ist die Haltevorrichtung meist als steifer C-Bogen ausgestaltet, an dessen einem Ende die Röntgenquelle und an dessen anderem Ende der Röntgendetektor derart angeordnet sind, dass sie sich direkt gegenüberliegen und Röntgenaufnahmen von einem dazwischen angeordneten Untersuchungsobjekt, z. B. einem Patienten, erstellt werden können. Mittels einer steifen Tragvorrichtung ist der C-Bogen dabei mit dem Raum, also dem Boden, der Decke oder der Wand, verbunden.

Weiter ist aus der WO 99/30614 eine C-Bogen-Röntgeneinrichtung bekannt, bei der die Tragvorrichtung als paralleler Manipulator ausgestaltet ist. Dabei ist eine erste an dem C-Bogen befestigte Halteplatte über mehrere Tragglieder mit einer zweiten Halteplatte verbunden, die beispielsweise an der Wand angebracht werden kann. Jedes dieser Tragglieder ist unmittelbar mit beiden Halteplatten verbunden, wobei die Verbindungspunkte der Tragglieder mit den Halteplatten verschieden sind. Damit läßt sich der C-Bogen in gewissen Grenzen in bestimmte Stellungen bringen.

Aus der US 4,894,855 ist eine Röntgeneinrichtung bekannt, bei der die Röntgenröhre, der Röntgendetektor und der Patiententisch an jeweils einem aus mehreren Traggliedern bestehenden Manipulator angebracht sind, die eine Bewegung dieser Elemente in allen drei räumlichen Richtungen unabhängig voneinander zulassen.

Als nachteilig erweist sich bei der aus der WO 99/30614 bekannten Lösung, dass die Flexibilität und die Bewegungsmöglichkeiten insbesondere aufgrund des mechanischen Aufbaus begrenzt sind. Für viele Anwendungen, beispielsweise für die 3D-Rotationsangiographie ist es jedoch gerade erforderlich, dass Röntgenaufnahmen aus verschiedensten Positionen erstellt werden können, dass beispielsweise eine Serie von Röntgenprojektionsaufnahmen entlang einer vorgegebenen Trajektorie erstellt werden kann. Dies muß auch mit einer möglichst hohen Positioniergenauigkeit erfolgen. Zwar ist bei der aus der US 4,894,855 bekannten Röntgeneinrichtung eine hohe Flexibilität gegeben; für viele Anwendungen ist die damit erreichbare Genauigkeit jedoch nicht ausreichend. Außerdem sind dort mehrere Manipulatoren erforderlich, die jeweils separat gesteuert werden müssen, was zu hohen Kosten führt und eine komplexe Steuerung erfordert.

Der Erfindung liegt deshalb die Aufgabe zugrunde, eine möglichst flexible Röntgeneinrichtung anzugeben, bei der eine hohe Positioniergenauigkeit gewährleistet ist und welche möglichst kostengünstig herzustellen ist.

Diese Aufgabe wird durch eine Röntgeneinrichtung gemäß Anspruch 1 gelöst.

Bei der Erfindung wurde erkannt, dass weder eine steife Tragvorrichtung noch ein paralleler Manipulator als Tragvorrichtung hinsichtlich der Flexibilität die optimale Lösung darstellen. Die Tragvorrichtung ist deshalb erfindungsgemäß aus mehreren gelenkig seriell miteinander verbundenen Traggliedern aufgebaut, die bevorzugt auch einzeln steuerbar sind, so dass die Haltevorrichtung in allen räumlichen Richtungen bewegt werden kann. Die erforderliche Positionsgenauigkeit wird auch dadurch erreicht, dass die Haltevorrichtung bevorzugt steif ausgebildet ist, so dass der Abstand zwischen Röntgenröhre und Röntgendetektor und die Ausrichtung dieser Elemente aufeinander unveränderbar sind. Außerdem ist hier nur die Tragvorrichtung aus mehreren Traggliedern aufgebaut, welche gesteuert werden muß, während bei der aus der US 4,894,855 bekannten Röntgeneinrichtung mehrere Manipulatoren vorhanden sind, die separat gesteuert werden müssen.

In einer bevorzugten Ausgestaltung der Erfindung ist vorgesehen, dass die Tragvorrichtung ein serieller Manipulator, insbesondere ein Roboterarm ist. Insbesondere kann auch ein aus vielen Bereichen der Produktionstechnik bekannter herkömmlicher serieller Roboterarm Verwendung finden. Dies kann zu einer Kostenreduzierung führen, da die Stückzahlen von C-Bogen-Röntgeneinrichtungen sehr begrenzt sind, so dass die Herstellungskosten der mechanischen Systeme einen deutlichen Kostenfaktor darstellen. Durch die Verwendung eines in großer Stückzahl hergestellten konventionellen Roboterarms lassen sich die Kosten für diesen Teil der Mechanik jedoch ggf. um einiges reduzieren bei gleichzeitiger Erhöhung der Bewegungsfreiheit. Die Steuerung derartiger Roboterarme ist mit bekannter und standardisierter Software auf einfache Weise möglich. Da derartige Roboterarme auch mit hoher Geschwindigkeit arbeiten, läßt sich ggf. auch eine Verkürzung der für die Erstellung von Röntgenaufnahmen erforderlichen Zeit realisierbar. Beispielsweise ließe sich ggf. in Echtzeit eine Studie des Verlaufs des Blutflusses erstellen.

Bei bevorzugten Weiterbildung ist vorgesehen, dass die Tragvorrichtung derart ausgestaltet ist, dass eine beliebige Positionierbarkeit der Röntgenquelle und des Röntgendetektors erreichbar sind und/oder dass die Bewegungen der einzelnen Tragglieder der Tragvorrichtung steuerbar sind. Damit die Röntgenröhre wie bei der Rotationsangiographie gefordert kreisförmige oder schraubenförmige Trajektorien beschreiben kann, ist in einer weiteren bevorzugten Ausgestaltung vorgesehen, dass die Tragvorrichtung mit der Haltevorrichtung mittels eines Drehgelenks verbunden ist.

Zwar ist bevorzugt vorgesehen, dass die Haltevorrichtung als steifer C-Bogen ausgestaltet ist, es kann jedoch auch vorgesehen sein, dass die Haltevorrichtung aus mindestens zwei Haltegliedern aufgebaut ist, wobei an einem ersten Halteglied die Röntgenquelle und an dem zweiten Halteglied der Röntgendetektor angeordnet sind. Dies hat den Vorteil, dass auch der Abstand zwischen Röntgenquelle und Röntgendetektor veränderbar ist, wodurch der Abbildungsmaßstab und die Größe des Untersuchungsbereichs verändert werden können und insgesamt die Flexibilität noch weiter erhöht wird.

Bekannte Robotersysteme weisen in der Regel ein mechanisches Notbremssystem auf, um die Bewegung des Roboters bei einer Störung oder Fehlsteuerung zu stoppen. Da bei medizinischen Anwendungen eine Verletzung eines Patienten in einem solchen Fall auf jeden Fall verhindert werden muß, sind bei einer bevorzugten Ausgestaltung Mittel vorgesehen zur Überwachung des Abstandes zwischen einem Untersuchungsobjekt und bewegten Teilen der Röntgeneinrichtung, insbesondere der Röntgenquelle und dem Röntgendetektor, wozu beispielsweise Ultraschallsensoren und Ultraschalldetektoren Verwendung finden können. Dadurch soll kontinuierlich der Abstand zwischen den bewegten Teilen und dem Untersuchungsobjekt gemessen werden und eine Notbremsung veranlaßt werden, sofern der Abstand zu gering wird und eine Verletzung des Untersuchungsobjekts drohen könnte. Der eine solche Notbremsung auslösende Abstand muß dabei jedoch so eingestellt sein, dass sich einerseits noch alle notwendigen Röntgenaufnahmen erstellen lassen und dass andererseits in dem verbleibenden Abstand noch eine Notbremsung möglich ist, bevor eine Berührung erfolgt.

Als weitere Mittel für die Abstandsüberwachung sind mechanische, an Röntgenquelle und Röntgendetektor angebrachte Berührungssensoren, z.B. fühlerartige Sensoren in der Form von langen Schnurbarthaaren, denkbar, durch bei Berührung mit dem Untersuchungsobjekt ein Sensorsignal liefern, wodurch eine Notbremsung auslösbar ist. Außerdem ist zur Überwachung auch eine separate Videoanlage denkbar, die kontinuierlich und in Echtzeit die Bewegung der Röntgenquelle und des Röntgendetektors beobachtet und auswertet und bei zu geringem Abstand eine Notbremsung einleitet.

Die Erfindung wird nachfolgend anhand der Zeichnungen näher erläutert. Es zeigen:
- Fig. 1: eine bekannte C-Bogen-Röntgeneinrichtung,
- Fig. 2: eine erfindungsgemäße C-Bogen-Röntgeneinrichtung,
- Fig. 3: eine weitere Ausführungsform einer erfindungsgemäßen Röntgeneinrichtung, und
- Fig. 4a, 4b: systematische Darstellungen einer weiteren Ausführungsform einer erfindungsgemäßen Röntgeneinrichtung in verschiedenen Positionen.

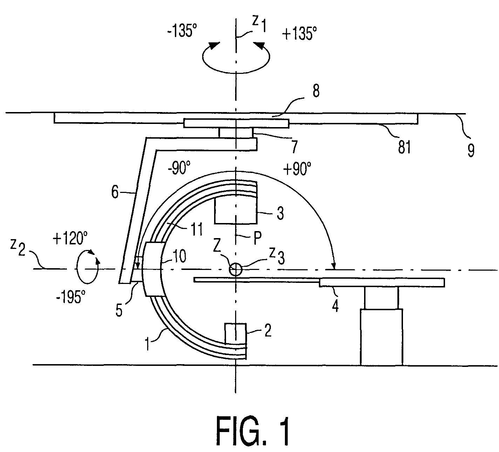

Die in Fig. 1 gezeigte bekannte Röntgeneinrichtung weist als Haltevorrichtung einen C-Bogen 1 mit einer Röntgenröhre 2 und einem Röntgendetektor 3 auf. Diese sind aufeinander ausgerichtet, so dass aus der Röntgenröhre 2 entlang des Projektionsstrahles P austretende Röntgenstrahlen ein im Untersuchungsbereich Z auf dem Patiententisch 4 angeordnetes Untersuchungsobjekt durchtritt und auf den Röntgendetektor 3 trifft. Mittels an dem C-Bogen 1 angebrachter Schienen 11, die durch eine Schienenhalterung 10 laufen, sind die Röntgenröhre 2 und der Röntgendetektor 3 im angegebenen Winkelbereich um die z₃-Achse rotierbar. Über ein Drehgelenk 5, das im gezeigten Fall eine Rotation um 315° um die z₂-Achse zuläßt, ist die Schienenhalterung 10 mit einer steifen Tragvorrichtung 6 verbunden. Diese ist wiederum mittels eines Gelenks 7, das eine Rotation um die z₁-Achse erlaubt, an einem Schlitten 8 angebracht, der in einem Schienensystem 81 läuft, das wiederum an der Decke 9 befestigt ist. Wie bereits durch die Winkelangaben deutlich wird, ist die Bewegungsfreiheit bei einer solchen Röntgeneinrichtung limitiert.

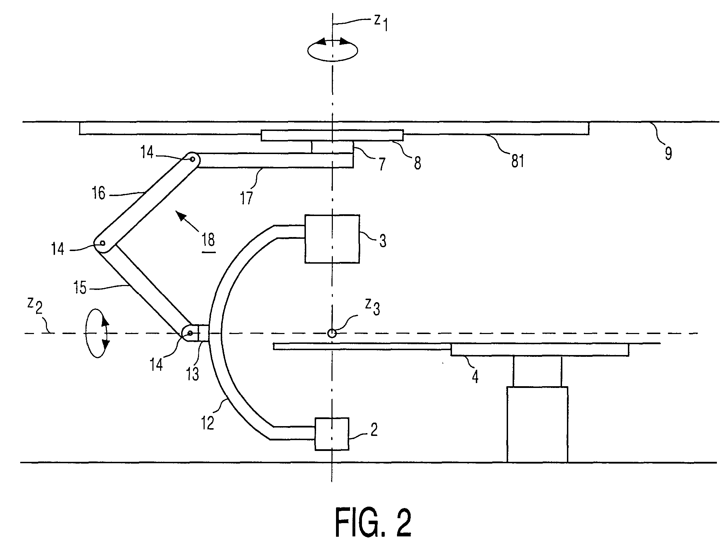

In Fig. 2 ist eine erste Ausführungsform einer erfindungsgemäßen Röntgeneinrichtung gezeigt. Dabei ist zunächst die Haltevorrichtung 12 wesentlich einfacher als steifer C-Bogen ohne Schienen ausgestaltet. Über ein Drehgelenk 13, das eine Rotation um 360° um die z₂-Achse zuläßt, ist die Haltevorrichtung 12 mit der Tragvorrichtung 18 verbunden. Diese umfaßt im gezeigten Fall drei Tragarme 15,16,17, die in Serie mittels einfacher ebener Gelenke 14 miteinander und an einem Ende mit dem Drehgelenk 7 und am anderen Ende mit dem Drehgelenk 13 verbunden sind. Die Gelenke 14 ermöglichen eine Veränderung der Lage der Haltevorrichtung 12 in der durch die Tragarme 15 und 16 aufgespannten Ebene. Die Befestigung an der Decke 9 erfolgt wie bei der in Fig. 1 gezeigten Röntgeneinrichtung. Durch die Ausgestaltung der Tragvorrichtung 18 läßt sich, wie leicht zu erkennen ist, die Haltevorrichtung 12 wesentlich flexibler positionieren. Zwar ist der Aufbau der Tragvorrichtung 18 im Vergleich zum Aufbau der Tragvorrichtung 6 (siehe Fig. 1) größer; dies wird jedoch dadurch ausgeglichen, dass hier die Haltevorrichtung 12 wesentlich einfacher, insbesondere ohne Schienen, und auch deutlich leichter ausgestaltet sein kann.

Um die Flexibilität einer solchen Röntgeneinrichtung weiter zu erhöhen, kann zudem vorgesehen sein, dass die Röntgenröhre 2 und/oder der Röntgendetektor 3 derart mittels einer Verschiebevorrichtung an dem C-Bogen 12 angebracht sind, dass sie in z₁-Richtung verschiebbar sind.

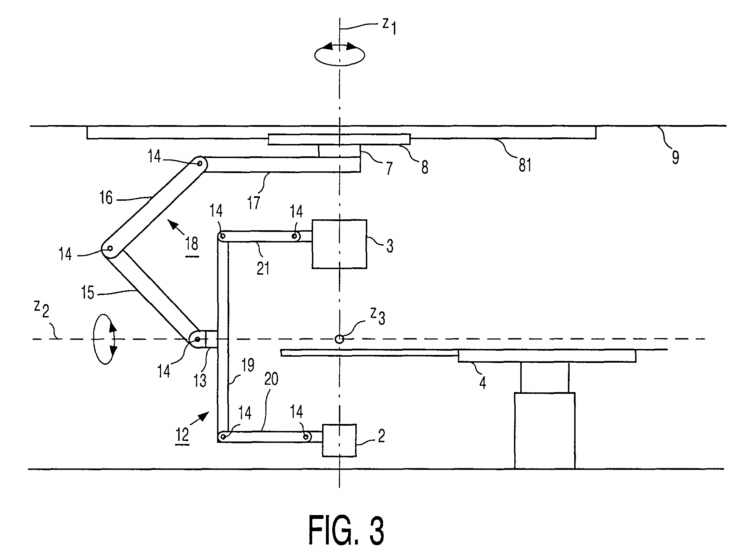

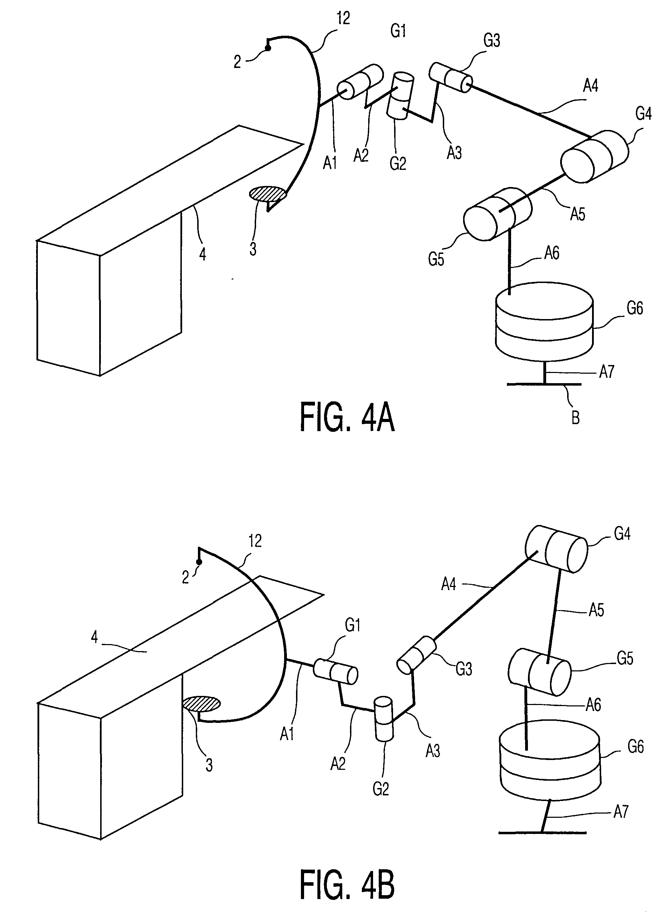

Eine weitere Ausführungsform einer erfindungsgemäßen Röntgeneinrichtung ist in Fig. 3 gezeigt. Bei dieser ist die Tragvorrichtung 18 wie bei der in Fig. 2 gezeigten Röntgeneinrichtung ausgestaltet, zusätzlich besteht jedoch auch die Haltevorrichtung 12 aus mehreren Haltegliedern 19,20,21. Diese Halteglieder bilden in etwa ein eckiges "C" und sind mittels ebener Gelenke 14 miteinander und mit der Röntgenröhre 2 bzw. dem Röntgendetektor 3 verbunden. Dadurch läßt sich auch der Abstand zwischen Röntgenröhre 2 und Röntgendetektor 3 und somit der Abbildungsmaßstab verändern.
Schematische Darstellungen einer weiteren Ausführungsform einer erfindungsgemäßen Röntgeneinrichtung, die sich in unterschiedlichen Positionen befindet, sind in den Figuren 4a und 4b gezeigt. Dabei erfolgt die Verbindung von der Haltevorrichtung 12, die wie in Fig. 2 gezeigt als einfacher C-Bogen gestaltet sein kann, bis hin zum Boden B durch sieben Verbindungsstücke A₁ bis A₇, die jeweils mittels eines Gelenks G₁ bis G₆ miteinander verbunden sind. Jedes dieser Gelenke G₁ bis G₆ ermöglicht eine Rotation um eine Rotationsachse, so dass bei dieser Ausführungsform die Tragvorrichtung sechs Achsen aufweist und die größtmögliche Flexibilität bietet.

Bei der Darstellung in Fig. 4a ist der C-Bogen 12 am Ende des Patiententisches 4 angeordnet, um beispielsweise ein Schichtbild des Kopfes eines Patienten durch Rotationsangiographie zu ermitteln. Bei der Darstellung in Fig. 4b ist der C-Bogen 12 in der Mitte des Patiententisches 4 angeordnet, um beispielsweise dort eine Röntgenaufnahme des Abdomen eines Patienten zu erstellen. Eine solche Röntgeneinrichtung läßt sich beispielsweise mittels eines konventionellen 6-Achsen-Knickarm-Roboters realisieren, wie er vielfach in der Produktionstechnik, z.B. in der Automobiltechnik, verwendet wird.

## Patentansprüche

1. Röntgeneinrichtung mit einer Röntgenquelle (2) und einem Röntgendetektor (3), welche an jeweils einem Ende einer gemeinsamen Haltevorrichtung (12) angeordnet sind, wobei die Haltevorrichtung (12) durch eine Tragvorrichtung (18) mit dem Raum (9) verbunden ist,
dadurch gekennzeichnet,
dass die Tragvorrichtung (18) aus mehreren gelenkig seriell miteinander verbundenen Traggliedern (15, 16, 17) aufgebaut ist.

2. Röntgeneinrichtung nach Anspruch 1,
dadurch gekennzeichnet,
dass die Tragvorrichtung (18) ein serieller Manipulator, insbesondere ein Roboterarm, ist.

3. Röntgeneinrichtung nach Anspruch 1,
dadurch gekennzeichnet,
dass die Tragvorrichtung (18) derart ausgestaltet und mit der Haltevorrichtung (12) verbunden ist, dass die Haltevorrichtung mit der Röntgenquelle (2) und dem Röntgendetektor (3) beliebig positionierbar ist.

4. Röntgeneinrichtung nach Anspruch 1,
dadurch gekennzeichnet,
dass die Bewegungen der einzelnen Tragglieder (15, 16, 17) der Tragvorrichtung (18) steuerbar sind.

5. Röntgeneinrichtung nach Anspruch 1,
dadurch gekennzeichnet,
dass die Tragvorrichtung (18) mit der Haltevorrichtung (12) mittels eines Drehgelenks (13) verbunden ist.

6. Röntgeneinrichtung nach Anspruch 1,
dadurch gekennzeichnet, dass die Haltevorrichtung (12) aus mindestens zwei Haltegliedern (19, 20, 21) aufgebaut ist, wobei an einem ersten Halteglied (20) die Röntgenquelle (2) und an einem zweiten Halteglied (21) der Röntgendetektor (13) angeordnet sind.

7. Röntgeneinrichtung nach Anspruch 1,
dadurch gekennzeichnet,
dass die Haltevorrichtung (12) ein C-Bogen ist.

8. Röntgeneinrichtung nach Anspruch 1,
dadurch gekennzeichnet,
dass Mittel vorgesehen sind zur Überwachung des Abstandes zwischen einem Untersuchungsobjekt und bewegten Teilen (2, 3, 12, 18) der Röntgeneinrichtung, insbesondere der Röntgenquelle (2) und dem Röntgendetektor (3).

9. Röntgeneinrichtung nach Anspruch 8,
dadurch gekennzeichnet,
dass die Mittel zur Abstandsüberwachung Ultraschallsensoren und Ultraschalldetektoren aufweisen.

10. Röntgeneinrichtung nach Anspruch 8,
dadurch gekennzeichnet,
dass die Mittel zur Abstandsüberwachung mechanische Berührungssensoren aufweisen.
